# EUROPÄISCHE PATENTSCHRIFT

(11) **EP 1 394 155 B1**
(45) Veröffentlichungstag und Bekanntmachung des Hinweises auf die Patenterteilung: **28.06.2006**
(21) Anmeldenummer: 03017811.5
(22) Anmeldetag: 05.08.2003
(51) Int. Cl.: C07D 307/83, C07D 307/82, A61K 31/343, A61P 9/06

(54) **Verfahren zur Herstellung von 5-Nitrobenzofuranen**
Process for the preparation of 5-Nitrobenzofurans
Procédé pour la préparation des 5-nitrobenzofurannes

(30) Priorität: 19.08.2002 DE 10237819
(43) Veröffentlichungstag der Anmeldung: 03.03.2004
(73) Patentinhaber: LANXESS Deutschland GmbH, 51369 Leverkusen (DE)
(72) Erfinder: Mägerlein, Wolfgang, Dr., 50733 Köln (DE)

(56) Entgegenhaltungen:
- EP-A- 0 471 609
- EP-A- 1 116 719
- WO-A-00/47207
- WO-A-01/28974
- WO-A-98/46561
- JP-A- 54 163 597
- JP-A- 2002 255 954
- US-A- 3 558 667
- US-A- 5 223 510
- US-A- 5 854 282
- ERLENMEYER EH ET AL: "Zur Kenntnis des 5-Aminocumarons" HELVETICA CHIMICA ACTA, VERLAG HELVETICA CHIMICA ACTA. BASEL, CH, Bd. 31, 1948, Seiten 75-77, XP002009857 ISSN: 0018-019X
- SPYROUDIS S ET AL: "PHENYLIODONIOPHENOLATES FROM 1,2-DIHYDROXYBENZENE DERIVATIVES" TETRAHEDRON, ELSEVIER SCIENCE PUBLISHERS, AMSTERDAM, NL, Bd. 50, Nr. 39, 1994, Seiten 11541-11552, XP002910906 ISSN: 0040-4020
- L. J. POWERS ET AL.: "Antibacterial Activity of Nitrobenzofuranes" J. MED. CHEM., Bd. 13, Nr. 6, 1970, Seiten 1102-1105, XP002257496
- A. MOORADIAN ET AL.: "The Preparation of O-Aryl Oximes and Their Conversion to Benzofuranes" J. HETEROCYCL. CHEM., Bd. 4, Nr. 3, 1967, Seiten 441-444, XP002257497
- CAGNIANT P ET AL: "CHIMIE ORGANIQUE. - CONTRIBUTION A L'ETUDE DU NAPHTO-(2.3-B) FURANNE ET DE SON ANALOGUE SELENIE LE NAPHTO-(2.3-B) SELENOPHENE NAPHTHO(2,3-B)FURAN AND ITS SELENIUM ANALOG NAPHTHO(2,3-B)SELENOHONE" COMPTES RENDUS HEBDOMADAIRES DES SEANCES DE L'ACADEMIE DES SCIENCES, SERIE C: SCIENCES CHIMIQUES, GAUTHIER-VILLARS. MONTREUIL, FR, Bd. 276, Nr. 22, 28. Mai 1973 (1973-05-28), Seiten 1629-1631, XP009019118

## Beschreibung

Die vorliegende Erfindung betrifft 5-Nitrobenzofurane, ein Verfahren zur Herstellung von 5-Nitrobenzofuranen sowie 5-Nitro-2,3-dihydro-benzofuran-3-ole, ein Verfahren zu deren Herstellung und Zwischenprodukte.

Benzofurane haben insbesondere als Zwischenprodukte für die Herstellung von Arzneimitteln technische Bedeutung erlangt. Die Benzofuran-Struktur findet sich beispielsweise in Wirkstoffen gegen Herzrhythmusstörungen, wie zum Beispiel Amiodaron und Bufuralol.

Weiterhin dient 2-(n-Butyl)-5-nitrobenzofuran als Zwischenprodukt bei der Herstellung von Dronedarone (N-[2-(n-Butyl)-3-[4-[3-(dibutylamino)propoxy]benzoyl]-5-benzofuranyl]-methan-sulfonamid), das ebenfalls gegen Herzrhythmusstörungen wirksam ist.

In EP-A 0 471 609 ist ein Verfahren zur Herstellung von 2-(n-Butyl)-5-nitrobenzofuran beschrieben, das von 2-Hydroxy-5-nitrobenzylbromid ausgeht. Dabei wird durch Umsetzung mit Triphenylphosphan zunächst 2-Hydroxy-5-nitrobenzyl-triphenylphosphoniumbromid hergestellt, das mit Pentanoylchlorid in Gegenwart einer Aminbase acyliert und anschließend zu 2-(n-Butyl)-5-nitrobenzofuran cyclisiert wird. Nachteilig an diesem Verfahren ist das teure Edukt 2-Hydroxy-5-nitrobenzylbromid, die geringe Ausbeute sowie die großen Abfallmengen insbesondere an Triphenylphosphanoxid.

Weiterhin sind aus WO-A 01/28974 und WO-A 01/29019 Verfahren zur Herstellung von 5-Nitrobenzofüranen bekannt, die ausgehend von Salicylaldehyd in einer vierstufigen Synthese über 2-(2-Formyl-4-nitrophenoxy)carbonsäuren als Zwischenprodukte verlaufen.

Nachteilig an diesem Verfahren ist der Preis für Salicylaldehyd, sowie die Tatsache, dass die oxidationsempfindliche Aldehyd-Funktionalität in allen Intermediaten enthalten ist.

Bei einem weiteren Verfahren gemäß EP-A 1 116 719 wird 5-Nitro-2(3H)-benzofuranon in Gegenwart von Pentansäure und Pentansäureanhydrid zu 3-(1-Hydroxypentyliden)-5-nitro-2(3H)-benzofuranon umgesetzt, das unter sauren Bedingungen zu 2-(n-Butyl)-5-nitrobenzofuran reagiert. Nachteilig an diesem Verfahren ist, dass als Edukt 5-Nitro-2(3H)-benzofuranon verwendet wird, was die technische Anwendung unwirtschaftlich werden lässt.

Es bestand daher weiterhin das Bedürfnis, ein effizientes und breit anwendbares Verfahren zur Herstellung von 5-Nitrobenzofuranen zu entwickeln, das sowohl von preisgünstigen, leicht erhältlichen Edukten ausgeht, als auch technisch in einfacher Weise realisierbar ist.

Es wurde nun ein Verfahren zur Herstellung von Verbindungen der Formel (I) gefunden, 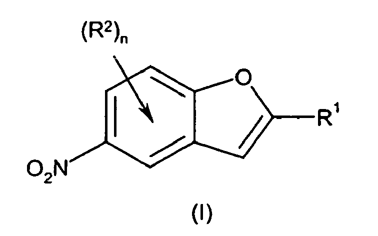 in der
- R¹ für Wasserstoff oder C₁-C₁₂-Alkyl steht und
- R² jeweils unabhängig stehen für: Fluor, Chlor, Brom, Iod, C₁-C₁₂-Alkyl, C₁-C₁₂₋Alkoxy, Hydroxy, NR³R⁴ oder CONR³R⁴, wobei R³ und R⁴ jeweils unabhängig voneinander für Wasserstoff oder C₁-C₁₂-Alkyl steht oder NR³R⁴ zusammen für einen cyclischen Aminorest mit 4 bis 12 Kohlenstoffatomen steht, COO-(C₁-C₁₂₋Alkyl), -COO(C₄-C₂₄-Aryl), -COO(C₅-C₂₅-Arylalkyl), CO(C₁-C₁₂-Alkyl), CO(C₄₋C₂₄-Aryl) oder C₁-C₁₂-Fluoralkyl und
- n für Null, eins, zwei oder drei steht oder für den Fall dass n zwei oder drei ist, zwei benachbarte Substituenten R² Teil eines annelierten Ringsystems sein können, das seinerseits gegebenenfalls mit den für R² vorstehend genannten Resten substituiert sein kann,
das dadurch gekennzeichnet ist, dass
Verbindungen der Formel (II), 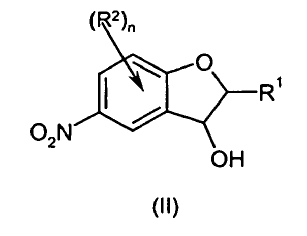 in der
R¹, R² und n die unter der Formel (I) genannte Bedeutung besitzen,
durch Dehydratisierung in Verbindungen der Formel (I) überführt werden

Im Rahmen der Erfindung können alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Weise kombiniert werden.

Alkyl beziehungsweise Alkoxy bedeutet jeweils unabhängig einen geradkettigen, cyclischen, verzweigten oder unverzweigten Alkyl- beziehungsweise Alkoxy-Rest. Gleiches gilt für den nichtaromatischen Teil eines Arylalkyl-Restes.

C₁-C₄-Alkyl steht beispielsweise für Methyl, Ethyl, n-Propyl, iso-Propyl, n-Butyl, sec.-Butyl und tert.-Butyl, C₁-C₈-Alkyl darüber hinaus beispielsweise für n-Pentyl, 1-Methylbutyl, 2-Methylbutyl, 3-Methylbutyl, neo-Pentyl, 1-Ethylpropyl, Cyclohexyl, Cyclopentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, 1-Ethyl-2-methylpropyl, n-Heptyl und n-Octyl, C₁-C₁₂-Alkyl weiter darüber hinaus beispielsweise für Adamantyl, die isomeren Menthyle, n-Nonyl, n-Decyl und n-Dodecyl.

C₁-C₄-Alkoxy steht beispielsweise für Methoxy, Ethoxy, n-Propoxy, iso-Propoxy, n-Butoxy, sec.-Butoxy und tert.-Butoxy, C₁-C₈-Alkoxy darüberhinaus für n-Pentoxy, 1-Methylbutoxy, 2-Methylbutoxy, 3-Methylbutoxy, neo-Pentoxy, 1-Ethylpropoxy, Cyclohexoxy, Cyclopentoxy, n-Hexoxy und n-Octoxy, C₁-C₁₂-Alkoxy weiter darüber hinaus beispielsweise für Adamantoxy, die isomeren Menthoxy-Reste, n-Decoxy und n-Dodecoxy.

**Fluoralkyl** bedeutet jeweils unabhängig einen geradkettigen, cyclischen, verzweigten oder unverzweigten Alkyl-Rest, der einfach, mehrfach oder vollständig durch Fluoratome substituiert ist.

Beispielsweise steht C₁-C₁₂-Fluoralkyl für Trifluormethyl, 2,2,2-Trifluorethyl, Pentafluorethyl, Nonafluorbutyl, Heptafluorisopropyl, Perfluoroctyl und Perfluordodecyl.

**Aryl** steht jeweils unabhängig für einen heteroaromatischen Rest mit 4 bis 24 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, vorzugsweise jedoch für einen carbocyclischen aromatischen Rest mit 6 bis 24 Gerüstkohlenstoffatomen.

Beispiele für carbocyclische aromatische Reste mit 6 bis 24 Gerüstkohlenstoffatomen sind Phenyl, Naphthyl, Phenanthrenyl, Anthracenyl oder Fluorenyl, heteroaromatische Reste mit 4 bis 24 Gerüstkohlenstoffatomen in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, sind beispielsweise Pyridinyl, Oxazolyl, Benzofuranyl, Dibenzofuran-yl oder Chinolinyl.

Weiterhin kann der carbocyclische aromatische Rest oder heteroaromatische Rest mit bis zu fünf gleichen oder verschiedenen Substituenten pro Cyclus substituiert sein, die ausgewählt sind aus der Gruppe Chlor, Fluor, C₁-C₁₂-Alkyl, C₁-C₁₂-Alkoxy, Di(C₁-C₈₋alkyl)amino, COO(C₁-C₈-Alkyl), CON(C₁-C₈-Alkyl)₂, COO(C₁-C₈-Arylalkyl), COO(C₄₋C₁₄-Aryl), CO(C₁-C₈-Alkyl), C₅-C₁₅-Arylalkyl oder Tri(C₁-C₆-alkyl)siloxyl.

C₄-C₂₄-Aryl steht beispielsweise und bevorzugt für Phenyl, o-, p-, m-Tolyl, o-, p-, m-Anisyl, o-, p-, m-Fluorphenyl, o-, p-, m-Chlorphenyl, o-, p-, m-Trifluormethylphenyl, o-, p-, m-Nitrophenyl und 2-, 3- und 4-Pyridyl.

**Arylalkyl** bedeutet jeweils unabhängig einen geradkettigen, cyclischem, verzweigten oder unverzweigten Alkyl-Rest nach obiger Definition, der einfach, mehrfach oder vollständig durch Aryl-Reste gemäß obiger Definition substituiert sein kann.

C₅-C₁₅-Arylalkyl steht beispielsweise und bevorzugt für Benzyl oder (R)- oder (S)-1-Phenylethyl.

Im Folgenden werden die bevorzugten Substitutionsmuster für Verbindungen der Formeln (I) und (II) definiert:
R¹ steht bevorzugt für Wasserstoff oder C₁-C₄-Alkyl und besonders bevorzugt für n-Butyl.
R² steht bevorzugt jeweils unabhängig für: Fluor, Chlor, C₁-C₄-Alkyl, C₁-C₄-Alkoxy, Hydroxy, COO-(C₁-C₄-Alkyl), Trifluormethyl, NR³R⁴ oder CONR³R⁴, wobei R³ und R⁴ jeweils unabhängig voneinander für Wasserstoff oder C₁-C₄-Alkyl stehen oder NR³R⁴ zusammen für Pyrrolidinyl oder Piperidinyl steht.
n steht bevorzugt für null oder eins, besonders bevorzugt für null.

Als Verbindung der Formel (I) ist ganz besonders bevorzugt 2-(n-Butyl)-5-nitrobenzofuran.

Weiterhin sind auch die Verbindungen der Formel (II) von der Erfindung erfasst. Als Verbindung der Formel (II) ist ganz besonders bevorzugt 2-(n-Butyl)-5-nitro-2,3-dihydrobenzofuran-3-ol. Dabei sind von der Erfindung bei den Verbindungen der Formel (II), sofern R¹ nicht für Wasserstoff steht, die (2R,3R)-; (2S,3S)-; (2S,3R)- und (2R,3S)-Isomeren jeweils in reiner Form als auch beliebige Mischungen der Isomere umfasst.

Im Rahmen der Erfindung sind als Dehydratisierungsreagentien, die gegebenenfalls in katalytischen Mengen eingesetzt werden können, besonders geeignet: Protonensäuren wie H₂SO₄ oder H₃PO₄ oder Hydrogensulfate wie KHSO₄, Hydroxide wie NaOH und KOH, P₂O₅, I₂ sowie Zink- und Kupfersalze wie insbesondere CuSO₄ und ZnCl₂. Für das erfindungsgemäße Verfahren besonders bevorzugt sind Protonensäuren. Für das erfindungsgemäße Verfahren ganz besonders bevorzugt ist H₂SO₄.

Die Dehydratisierung kann in einem organischen Lösungsmittel durchgeführt werden. Als organische Lösungsmittel werden bevorzugt polare, protische und aprotische Lösungsmittel verwendet. Unter polaren Lösungsmitteln sind solche zu verstehen, die eine Dielektrizitätskonstante bei 25°C von 5 oder mehr aufweisen. Für das erfindungsgemäße Verfahren besonders bevorzugte organische Lösungsmittel sind aliphatische C₁-C₆-Alkohole. Für das erfindungsgemäße Verfahren ganz besonders bevorzugt ist Ethanol.

Die Dehydratisierung kann beispielsweise bei Temperaturen von -20 bis 150°C durchgeführt werden, bevorzugt bei 20 bis 150°C, besonders bevorzugt bei 50 bis 100°C und ganz besonders bevorzugt bei 70 bis 78°C.

Die Reaktionsdauer kann beispielsweise 0.5 bis 20 Stunden, bevorzugt 0.5 bis 8 Stunden und besonders bevorzugt 4 bis 5 Stunden betragen.

Der Druck bei der Reaktion ist unkritisch und kann beispielsweise 0.5 bis 100 bar, bevorzugt 0.8 bis 10 bar betragen. Besonders bevorzugt ist Umgebungsdruck.

Die Verbindungen der Formel (II) können in besonders vorteilhafter Weise durch Reduktion von Verbindungen der Formel (III) erhalten werden, 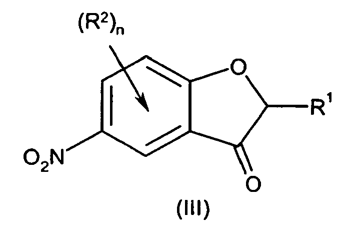 in der

R¹, R² und n die unter der Formel (I) genannte Bedeutung einschließlich der Vorzugsbereiche besitzen.

Als Verbindungen der Formel (III) sind solche von der Erfindung umfasst, in denen R² und n die unter der Formel (I) genannten Bedeutungen einschließlich der Vorzugsbereiche besitzen und R¹ für n-Butyl steht. Eine besonders bevorzugte Verbindung der Formel (III) ist 2-(n-Butyl)-5-nitro-3(2H)-benzofuranon.

Die Reduktion der Verbindungen der Formel (III) kann beispielsweise durch Transferhydrierung oder mittels Aluminium-Wasserstoff- oder Bor-Wasserstoff-Verbindungen erfolgen. Bevorzugt erfolgt die Reduktion durch Aluminium-Wasserstoff- oder Bor-Wasserstoff-Verbindungen, besonders bevorzugt, durch Verbindungen der Formel (RI)

Met[EH_{q}R⁵_{(4-q)}]ₚ (RI)

in der
- Met: für ein ein- oder zweiwertiges Metall wie vorzugsweise Zink, Lithium, Natrium oder Kalium steht und
- E: für Aluminium oder Bor und
- R⁵: für C₁-C₈-Alkyl steht und
- q: für 1,2,3 oder 4, bevorzugt für 4 oder 1 und
- p: für die Wertigkeit von Met steht
oder durch Verbindungen der Formel (RII)

BHᵣR⁵₍₃₋ᵣ₎ (RII)

in der
- R⁵: die unter Formel (RI) genannte Bedeutung besitzt und
- r: für eins, zwei oder drei steht.

Ganz besonders bevorzugte Verbindungen der Formeln (RI) und (RII) sind LiBH₄, NaBH₄, Zn(BH₄)₂, LiAlH₄, Li[BHEthyl₃] und Li[AlH(sek.-butyl)₃], wobei NaBH₄ noch weiter bevorzugt ist.

Die Menge der Aluminium-Wasserstoff- oder Bor-Wasserstoff-Verbindungen wird vorzugsweise so gewählt, dass das molare Verhältnis von Wasserstoff in den Aluminium-Wasserstoff- oder Bor-Wasserstoff-Verbindungen und dem Substrat 0.5 bis 10 beträgt, bevorzugt 0.95 bis 5 und besonders bevorzugt 1.0 bis 1.2. Größere Verhältnisse sind möglich aber unwirtschaftlich.

Als Lösungsmittel können, je nach eingesetztem Reagenz, polare Lösungsmittel eingesetzt werden. Für das erfindungsgemäße Verfahren bevorzugt ist der Einsatz von Alkalimetallboranaten in aliphatischen C₁-C₆-Alkoholen oder Ethern wie insbesondere 1,4-Dioxan. Ganz besonders bevorzugt wird die Reduktion mit Natriumborhydrid in Ethanol durchgeführt.

Die Reaktion kann beispielsweise bei Temperaturen von -78 bis 100°C durchgeführt werden, bevorzugt 0 bis 100°C und besonders bevorzugt 60 bis 78°C.

In besonders vorteilhafter Weise lassen sich die Verbindungen der Formel (III) durch Nitrierung von Verbindungen der Formel (IV) erhalten, 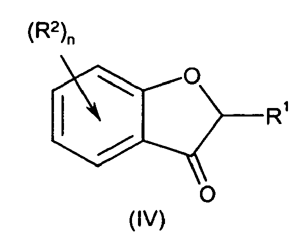 in der R¹, R² und n die unter der Formel (I) genannten Bedeutungen einschließlich der Vorzugsbereiche besitzen.

Zur Nitrierung werden vorteilhafterweise solche Reagentien eingesetzt werden, die Nitryl-Kationen (NO₂⁺) erzeugen können. Bevorzugt ist beispielsweise Salpetersäure, vorzugsweise mit einem Gehalt von 60 bis 100 Gew.-% bezogen auf HNO₃. Der Einsatz von Salpetersäure gegebenenfalls als Mischung mit Schwefelsäure mit einem Gehalt von 50 bis 160 Gew.-% bezogen auf H₂SO₄ und/oder Phosphorsäure mit einem Gehalt von 70 bis 160 Gew.-% bezogen auf H₃PO₄ ist ebenso bevorzugt. Weiterhin bevorzugt ist der Einsatz von Stickstoffoxiden wie NO₂, N₂O₃, N₂O₄, N₂O₅ oder von NO, gegebenenfalls in Anwesenheit eines Oxidationsmittels. Ebenfalls bevorzugt ist die Nitrierung mit salpetriger Säure HNO₂ oder einem Salz der salpetrigen Säure wie insbesondere NaNO₂ vorzugsweise in Anwesenheit eines Oxidationsmittels oder mit ionischen Nitrylverbindungen, wie zum Beispiel NO₂⁺BF₄⁻. Ferner sind bevorzugt zur Nitrierung Alkylnitrite der allgemeinen Formel R⁶-ONO einzusetzen, wobei R⁶ für C₁-C₁₂-Alkyl steht.

Die eingesetzte Menge des Nitrierungsreagenzes wird vorteilhafterweise so gewählt, dass das molare Verhältnis von theoretisch nitrierendem Agens zu Verbindung der Formel (IV) 0.9 bis 2 beträgt, bevorzugt 1.0 bis 1.5 und besonders bevorzugt 1.0 bis 1.2.

Besonders bevorzugt wird für die Nitrierung eine Mischung aus Salpetersäure mit einem Gehalt von 60 bis 100 Gew.-% bezogen auf HNO₃ und Schwefelsäure mit einem Gehalt von 90 bis 100 Gew.-% bezogen auf H₂SO₄ eingesetzt.

Ebenso ist für die Nitrierung Salpetersäure mit einem Gehalt von 95 bis 100 Gew.-% besonders bevorzugt.

In einer bevorzugten Ausführungsform liegt das molare Verhältnis von Verbindung der Formel (IV) und Salpetersäure zwischen 0.9 und 1, besonders bevorzugt zwischen 0.95 und 1.05.

Die Nitrierung kann beispielsweise mit oder ohne organischem Lösungsmittel durchgeführt werden, wobei das organische Lösungsmittel unter den Reaktionsbedingungen inert sein soll. Besonders erwähnt seien als Vertreter für derartige organische Lösungsmittel halogenierte aliphatische Kohlenwasserstoffe wie insbesondere chlorierte aliphatische Kohlenwasserstoffe wie zum Beispiel Dichlormethan, 1,2-Dichlorethan, Tetrachlormethan und Hexachlorethan. Für die Nitrierung von Verbindungen der Formel (IV) bevorzugt ist Dichlormethan.

Die Nitrierung wird bevorzugt jedoch ohne organisches Lösungsmittel durchgeführt.

Die Stoffmengenkonzentration der Verbindung der Formel (IV) im Reaktionsmedium kann beispielsweise 0.2 bis 3 mol/l betragen, bevorzugt ist 0.3 bis 1.6 mol/l.

Die Nitrierung kann beispielsweise in einem Temperaturbereich von -10 bis 50°C durchgeführt werden, bevorzugt zwischen -10 und 20°C, besonders bevorzugt zwischen -5 und 10°C.

Das erfindungsgemäße Verfahren kann unter einer Inertgasatmosphäre bei Normaldruck durchgeführt werden, wobei beispielsweise folgendermaßen vorgegangen werden kann: Zum Beispiel kann die Schwefelsäure vorgelegt werden und dann die Verbindung der Formel (IV) und die Salpetersäure oder eine Mischung aus Salpetersäure und Schwefelsäure zudosiert werden. Es kann femer auch die Schwefelsäure und die Salpetersäure vorgelegt und dann, vorzugsweise in Portionen, die Verbindung der Formel (IV) zugesetzt werden.

Die Reaktionsdauer kann je nach eingesetzter Menge beispielsweise und bevorzugt 0.1 bis 10 Stunden betragen.

Zur Isolierung des Produktes können die bei Nitrierungsreaktionen üblichen, dem Fachmann bekannten Techniken angewandt werden. Für das erfindungsgemäße Verfahren bevorzugt ist ein Überführen der Reaktionsmischung auf Eis oder Eiswasser und anschließende Filtration oder Extraktion.

In bevorzugter Weise können die Verbindungen der Formel (IV) durch Hydrolyse von Verbindungen der Formel (V) erhalten werden, 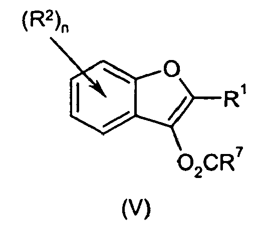 in der
- R¹, R²: und n die unter der Formel (I) genannte Bedeutung einschließlich der Vorzugsbereiche besitzen und
- R⁷: für C₁-C₁₂-Alkyl, C₅-C₂₅-Arylalkyl, C₄-C₂₄-Aryl oder C₁-C₁₂-Fluoralkyl steht.
- R⁷: steht bevorzugt für C₁-C₄-Alkyl und besonders bevorzugt für Methyl.

Die Hydrolyse kann dabei durch Säuren, Basen, Metallionen, Enzyme oder Nukleophile, gegebenenfalls in katalytischen Mengen und gegebenenfalls in Gegenwart eines organischen Lösungsmittels durchgeführt werden.

Vorzugsweise werden Basen wie beispielsweise wässrige Amine, wie insbesondere wässriges Ammoniak, Hydroxide oder Carbonate wie insbesondere NaOH und KOH oder Säuren wie beispielsweise wässrige Salzsäure, Schwefelsäure, Phosphorsäure, Hydrogensulfate, Carbonsäuren wie beispielsweise Ameisensäure oder Essigsäure, sowie Sulfonsäuren, wie zum Beispiel Methansulfonsäure oder *para*-Toluolsulfonsäure in unterschiedlichen Konzentrationen eingesetzt.

Besonders bevorzugt werden Säuren eingesetzt, ganz besonders bevorzugt Salzsäure, Schwefelsäure oder Phosphorsäure, wobei 6N Salzsäure noch weiter bevorzugt ist.

Die Säure wird dabei vorzugsweise in solchen Mengen eingesetzt, dass das molare Verhältnis von Verbindungen der Formel (V) zu Säure 1:1 bis 1:100, bevorzugt 1:1,1 bis 1:10 und besonders bevorzugt 1:5 bis 1:6 beträgt.

In einer bevorzugten Ausführungsform kann zur Verbesserung der Löslichkeit der Verbindungen der Formel (V) ein organisches Lösungsmittel eingesetzt werden. Weiterhin ist auch intensives Rühren sowie die Anwendung von Ultraschall oder von Phasentransferkatalysatoren möglich. Als organische Lösungsmittel bevorzugt sind aliphatische C₁-C₆-Alkohole oder cyclische Ether. Besonders bevorzugt wird Ethanol eingesetzt.

Die Reaktion kann bei Temperaturen von 25 bis 200°C durchgeführt werden, bevorzugt bei 60 bis 100°C, besonders bevorzugt bei 70 bis 78°C.

Die Reaktionsdauer kann beispielsweise 0.5 bis 24 Stunden betragen, bevorzugt sind 1 bis 6 Stunden.

Eine besonders bevorzugte Verbindung der Formel (V) ist 3-Acetoxy-2-(n-butyl)-benzofuran.

Die Verbindungen der Formel (V) können in besonders vorteilhafter Weise durch Cyclisierung-Decarboxylierung von Verbindungen der Formel (VI), 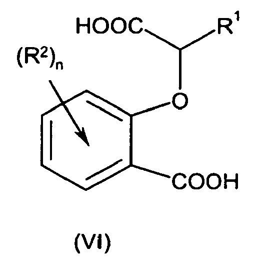 in der
R¹, R² und n die unter der Formel (I) genannte Bedeutung einschließlich der Vorzugsbereiche besitzen,
in Gegenwart mindestens einer Verbindung der Formel (RIII)

R⁷COR⁸ (RIII)

in der
- R⁷: die unter der Formel (V) genannte Bedeutung einschließlich der Vorzugsbereiche besitzt und
- R⁸: für -O₂CR⁷, Hydroxy oder OM steht, wobei M für ein Erdalkali- oder Alkalimetall steht,
erhalten werden.

Die Reaktion wird bevorzugt in Gegenwart einer Mischung von Verbindungen der Formel (RIII) durchgeführt, wobei jeweils ein Anhydrid, vorzugsweise ein Homoanhydrid (R⁷CO)₂O, ein Alkalimetallsalz und eine freie Säure eingesetzt wird. Vorzugsweise sind in den genannten Verbindungen die Reste R⁷ jeweils identisch. Besonders bevorzugt werden Mischungen von Natriumacetat, Essigsäure und Essigsäureanhydrid eingesetzt. Bevorzugt ist dabei ein Stoffmengenverhältnis von (0,8 bis 1,5):(2 bis 4):(3 bis 8), besonders bevorzugt von etwa 1:3:5.

Die Reaktionstemperatur kann beispielsweise 20 bis 150°C betragen, bevorzugt 60 bis 150°C und besonders bevorzugt 120 bis 140°C.

In bevorzugter Weise können die Verbindungen der Formel (VI) durch Hydrolyse von Verbindungen der Formel (VII) erhalten werden 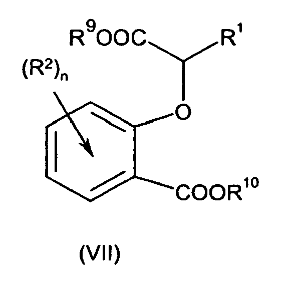 in der
- R¹, R² und n: die unter der Formel (I) genannte Bedeutung einschließlich der Vorzugsbereiche besitzen und
- R⁹ und R¹⁰: jeweils unabhängig voneinander für C₁-C₁₂-Alkyl, C₅-C₂₅-Arylalkyl oder C₄₋C₂₄-Aryl stehen und maximal ein Rest R⁹ oder R¹⁰ für Wasserstoff stehen kann.

Bevorzugt stehen R⁹ und R¹⁰ jeweils unabhängig voneinander für C₁-C₄-Alkyl, besonders bevorzugt jeweils unabhängig voneinander für Methyl oder Ethyl und ganz besonders bevorzugt jeweils identisch für Methyl oder Ethyl.

Die Hydrolyse kann dabei durch Säuren, Basen, Metallionen, Enzyme oder Nucleophile, gegebenenfalls in katalytischen Mengen und gegebenenfalls in Gegenwart eines organischen Lösungsmittels durchgeführt werden.

Vorzugsweise werden Basen wie beispielsweise wässrige Amine wie insbesondere wässriges Ammoniak, Hydroxide wie insbesondere LiOH, NaOH, KOH und Ca(OH)₂, Carbonate wie Na₂CO₃, K₂CO₃ oder CaCO₃ oder wässrige Säuren wie beispielsweise Salzsäure, Schwefelsäure, Phosphorsäure, Hydrogensulfate, Carbonsäuren wie beispielsweise Ameisensäure oder Essigsäure, sowie Sulfonsäuren, wie zum Beispiel Methansulfonsäure oder *para*-Toluolsulfonsäure eingesetzt.

In einer bevorzugten Ausführungsform wird die Hydrolyse in Gegenwart von Basen durchgeführt, wobei der Einsatz von LiOH, NaOH, KOH oder Ca(OH)₂ bevorzugt ist. Besonders bevorzugt ist der Einsatz von Natronlauge mit einem Gehalt von 5 bis 25 Gew.-%. Weiterhin kann in einer bevorzugten Ausführungsform zur Verbesserung der Löslichkeit der Verbindungen der Formel (VII) ein organisches Lösungsmittel eingesetzt werden.

Zur effizienten Verseifung der Estergruppen wird die Base vorzugsweise in solchen Mengen eingesetzt, dass das molare Verhältnis von Base zu Verbindungen der Formel (VII) 1,5:1 bis 20:1, bevorzugt 2:1 bis 7:1 und besonders bevorzugt 3:1 bis 5:1 beträgt.

Zur Verbesserung der Löslichkeit des Substrates kann ein organisches Lösungsmittel eingesetzt werden. Weiterhin ist intensives Rühren sowie die Anwendung von Ultraschall oder von Phasentransferkatalysatoren möglich. Als organische Lösungsmittel werden für das erfindungsgemäße Verfahren bevorzugt aliphatische C₁-C₆-Alkohole oder cyclische Ether verwendet. Besonders bevorzugt wird Methanol oder Ethanol eingesetzt.

Die Reaktion kann bei Temperaturen von 20 bis 200°C, vorzugsweise bei 40 bis 75°C und besonders bevorzugt bei 40 bis 60°C durchgeführt werden.

Als Verbindungen der Formel (VII) seien genannt:

2-(1-Methoxycarbonyl -pentoxy)-benzoesäuremethylester, 2-(1-Methoxycarbonylpentoxy)-benzoesäureethylester, 2-(1-Ethoxycarbonyl-pentoxy)-benzoesäureethylester und 2-(1-Ethoxycarbonyl-pentoxy)-benzoesäuremethylester, 2-(1-Methoxycarbonyl-pentoxy)-benzoesäure, 2-(1-Ethoxycarbonyl-pentoxy)-benzoesäure, 2-(1-Carboxy-pentoxy)-benzoesäureethylester und 2-(1-Carboxy-pentoxy)-benzoesäuremethylester, sowie 2-(1-Methoxycarbonyl-hexoxy)-benzoesäuremethylester, 2-(1-Methoxycarbonyl-hexoxy)-benzoesäureethylester, 2-(1-Ethoxycarbonyl-hexoxy)-benzoesäureethylester und 2-(1-Ethoxycarbonyl-hexoxy)-benzoesäuremethylester, 2-(1-Methoxycarbonyl-hexoxy)-benzoesäure, 2-(1-Ethoxycarbonyl-hexoxy)-benzoesäure, 2-(1-Carboxy-hexoxy)-benzoesäureethylester und 2-(1-Carboxy-hexoxy)-benzoesäuremethylester.

In bevorzugter Weise können die Verbindungen der Formel (VII) durch Umsetzung von Verbindungen der Formel (VIII), 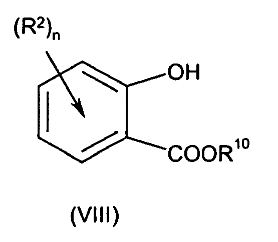 in der
- R² und n: die unter der Formel (I) genannten Bedeutungen einschließlich der Vorzugsbereiche besitzen und
- R¹⁰: für Wasserstoff, C₁-C₁₂-Alkyl, C₅-C₂₅-Arylalkyl oder C₄-C₂₄-Aryl steht
mit Verbindungen der Formel (IX) erhalten werden, 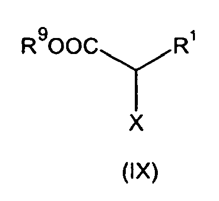 in der
- R¹: die unter der Formel (I) genannten Bedeutungen einschließlich der Vorzugsbereiche besitzt
- R⁹: für Wasserstoff, C₁-C₁₂-Alkyl, C₅-C₂₅-Arylalkyl oder C₄-C₂₄-Aryl steht und
- X: für Chlor, Brom, Iod oder R¹¹SO₃- steht, wobei R¹¹ für C₁-C₁₂-Alkyl, C₄-C₂₄- Aryl, C₅-C₂₅-Arylalkyl oder C₁-C₁₂-Fluoralkyl steht.

Vorzugsweise steht R¹¹ für C₁-C₄-Alkyl oder C₄-C₂₄-Aryl, besonders bevorzugt für Methyl, Ethyl, Phenyl, o-, m- oder p-Tolyl oder Trifluormethyl.

Bevorzugt steht X für Chlor oder Brom, besonders bevorzugt für Brom.

Als Verbindungen der Formel (VIII) werden bevorzugt o-Hydroxybenzoesäuremethylester und o-Hydroxybenzoesäureethylester eingesetzt.

Als Verbindungen der Formel (IX) werden bevorzugt die 2-Bromcarbonsäuremethylester oder -ethylester wie insbesondere 2-Bromhexansäuremethylester oder -ethylester eingesetzt.

Die Umsetzung von Verbindungen der Formel (VIII) mit Verbindungen der Formel (IX) zu Verbindungen der Formel (VII) wird vorzugsweise in Gegenwart von Base durchgeführt. Alternativ dazu kann die Verbindung der Formel (VIII) zunächst in das entsprechende Phenolat überführt werden und als solches in der Reaktion eingesetzt werden.

Die Reaktion kann in einem organischen Lösungsmittel durchgeführt werden. Ferner ist eine Reaktion auch in Wasser in Gegenwart eines Phasentransferkatalysators möglich. Für das erfindungsgemäße Verfahren werden bevorzugt polare protische oder aprotische Lösungsmittel eingesetzt, wie zum Beispiel Ketone wie Aceton, Amide wie N,N-Dimethylformamid oder N,N-Dimethylacetamid, Lactame wie 1-Methylpyrrolidin-2-on, Ether wie Tetrahydrofuran oder Dioxan, Nitrile wie Acetonitril oder Benzonitril oder Alkohole, wie Methanol oder Ethanol. Noch weiter bevorzugt für das erfindungsgemäße Verfahren sind Aceton oder Acetonitril.

Gegebenenfalls kann die Reaktion unter einer Inertgasatmosphäre, z.B. einer Argonatmosphäre, durchgeführt werden.

Als Basen werden solche eingesetzt, die die Verbindungen der Formel (VIII) an der Phenolfunktion zumindest teilweise deprotonieren können.

Es können beispielsweise anorganische Basen oder organische Basen eingesetzt werden. Als organische Basen können bevorzugt tertiäre Amine wie Triethylamin oder Alkalimetall-alkoholate wie Natriummethylat oder -ethylat verwendet werden, als anorganische Basen beispielsweise Alkali- oder Erdalkalimetallhydroxide, -carbonate oder -hydrogencarbonate. Bevorzugte Basen sind Alkalimatallcarbonate, besonders Kaliumcarbonat oder Natriumcarbonat.

Das Stoffmengenverhältnis der eingesetzten Verbindungen der Formel (IX) und der Verbindungen der Formel (VIII) wird vorteilhafterweise so gewählt, dass es zwischen 1.0 und 1.2 liegt.

Das Stoffmengenverhältnis zwischen der eingesetzten Base und der eingesetzten Verbindung der Formel (VIII) wird vorteilhafterweise zwischen 1.0 und 1.5 gewählt, wobei ein Verhältnis zwischen 1.1 und 1.3 bevorzugt ist.

Die Reaktion kann beispielsweise bei Temperaturen von 0 bis 150°C durchgeführt werden, bevorzugt sind Reaktionstemperaturen von 50 bis 100°C, besonders bevorzugt 70 bis 80°C.

Gegebenenfalls können die Verbindungen der allgemeinen Formel (VI) auch direkt, ohne Isolierung der Verbindungen der Formel (VII), durch Umsetzung von Verbindungen der Formel (VIII) mit Verbindungen der Formel (IX) mit gleichzeitig stattfindender Hydrolyse der Esterfunktionen in einem Eintopfverfahren hergestellt werden.

Verbindungen der Formel (VI) können alternativ auch so hergestellt werden, dass solche Verbindungen der Formel (VIII) mit solchen Verbindungen der Formel (IX) umgesetzt werden, in denen R⁹ und R¹⁰ jeweils für Wasserstoff stehen. Bei dieser Umsetzung wird die Basenmenge vorteilhafterweise entsprechend erhöht.

Die Erfindung umfasst neben einem Verfahren zur Herstellung von Verbindungen der Formel (I) aus Verbindungen der Formel (II) auch Verfahren zur Herstellung von Verbindungen der Formel (I) aus Verbindungen der Formel (III), (IV), (V), (VI), (VII), (VIII) und (IX), die jeweils über die beschriebenen Zwischenstufen verlaufen.

Weiterhin umfasst die Erfindung Verfahren zur Herstellung von Verbindungen der Formel (II) aus Verbindungen der Formel (III) sowie jeweils aus den Verbindungen (IV), (V), (VI), (VII), (VIII) und (IX), die jeweils über die beschriebenen Zwischenstufen verlaufen.

Weiterhin umfasst die Erfindung Verfahren zur Herstellung von Verbindungen der Formel (III) aus Verbindungen der Formel (IV) sowie jeweils aus den Verbindungen (V), (VI), (VII), (VIII) und (IX), die jeweils über die beschriebenen Zwischenstufen verlaufen.

Die erfindungsgemäß hergestellten Verbindungen der Formeln (I), (II) und (III) eignen sich insbesondere zur Herstellung von Arzneimitteln und physiologisch aktiven Stoffen und zur Anwendung in einem Verfahren zur Herstellung von Arzneimitteln und physiologisch aktiven Stoffen.

Bevorzugte Arzneimittel und physiologisch aktive Stoffe sind dabei solche, die zur Behandlung von Herzrhythmusstörungen eingesetzt werden. Ein besonders bevorzugter physiologisch aktiver Stoff ist Dronedarone.

Die Erfindung zeichnet sich dadurch aus, dass 5-Nitrobenzofurane, 5-Nitro-2,3-dihydrobenzofuran-3-ole und 5-Nitro-3(2H)-benzofuranone über einen hocheffizienten und ökonomischen Weg zugänglich sind und Syntheseverfahren für ein breites Spektrum an potentiellen Entwicklungskandidaten eröffnet werden.

### Beispiele:

### Beispiel 1: Synthese von 2-(1-Methoxycarbonyl-pentoxy)-benzoesäuremethylester

In einem 250 ml-Kolben wurden 15.2 g Salicylsäuremethylester und 18.0 g Kaliumcarbonat in 125 ml Acetonitril unter einer Argonatmosphäre vorgelegt und bei Raumtemperatur 20.9 g 2-Bromhexansäuremethylester zugegeben. Die farblose Suspension wurde 16 Stunden lang unter Rückfluss und Rühren erhitzt und der Reaktionsfortschritt durch Dünnschichtchromatographie kontrolliert. Nach Abkühlen auf Raumtemperatur wurde die Suspension filtriert und der Rückstand mit Aceton gewaschen. Nach Einengen des Filtrats im Vakuum wurden 27.8 g (99 % d. Th.) 2-(1-Methoxycarbonyl-pentoxy)-benzoesäuremethylester als gelbes Öl erhalten.

### Beispiel 2: Synthese von 2-(1-Carboxypentoxy)-benzoesäure

In einem 250 ml-Kolben wurden 13.0 g 2-(1-Methoxycarbonyl-pentoxy)-benzoesäuremethylester in Methanol gelöst und eine Lösung von 9.1 g Natriumhydroxid in 40 ml Wasser zugegeben. Das Reaktionsgemisch wurde bei 40°C eine Stunde lang gerührt; wobei sich ein Niederschlag bildete. Dieser wurde nach Abkühlen auf Raumtemperatur durch Zugabe von weiterem Methanol und Natronlauge in Lösung gebracht. Das Reaktionsgemisch wurde mit Dichlormethan gewaschen und die wässrige Phase anschließend unter Eiskühlung mit konzentrierter Salzsäure auf einen pH-Wert von 0 gebracht. Dabei fiel ein farbloser Feststoff aus, der abfiltriert und getrocknet wurde. Auf diese Weise wurden 10.1 g (87 % d. Th.) 2-(1-Carboxypentoxy)-benzoesäure erhalten.

### Beispiel 3: Synthese von 3-Acetoxy-2-(n-butyl)-benzofuran

In einem 25 ml-Kolben wurden 5.0 g 2-(1-Carboxypentoxy)-benzoesäure und 1.6 g Natriumacetat vorgelegt und mit 3.4 ml Eisessig und 9.4 ml Essigsäureanhydrid versetzt. Die farblose Suspension wurde 4 Stunden lang auf Rückfluss erhitzt. Nach Abkühlen, Überführen des Reaktionsgemisches auf Eiswasser, Extraktion mit Dichlormethan, Trocknen der vereinigten organischen Phasen über Natriumsulfat und Entfernen des Lösungsmittels erhielt man ein gelbes Öl, in dem laut GC das Edukt und das Produkt in einem ungefähren Verhältnis (Flächenprozente) von 50:50 vorhanden waren.

Dieses Öl wurde nochmals mit 1.6 g Natriumacetat, 3.4 ml Eisessig und 9.4 ml Essigsäureanhydrid versetzt und wiederum 4 Stunden lang auf Rückfluss erhitzt. Nach Abkühlen und gleicher Aufarbeitung wie oben beschrieben, erhielt man 4.3 g (92 % d. Th.) 3-Acetoxy-2-(n-butyl)-benzofuran als hellgelbes Öl.

### Beispiel 4: Synthese von 2-(n-Butyl)-3(2H)-benzofuranon

In einem 100 ml-Kolben wurden 4.6 g 3-Acetoxy-2-(n-butyl)-benzofuran in 20 ml Ethanol gelöst und 20 ml 6 N Salzsäure zugegeben. Es wurde 4 Stunden lang auf Rückfluss erhitzt. Nach dem Abkühlen auf Raumtemperatur wurde mit Natriumhydroxid ein pH-Wert von 4 bis 6 eingestellt und das Ethanol abdestilliert. Danach wurde mit Dichlormethan extrahiert. Nach Trocknen der vereinigten organischen Phasen über Natriumsulfat und Entfernen des Lösungsmittels erhielt man 2.8 g (76 % d. Th.) 2-(n-Butyl)-3 (2H)-benzofuranon als gelbes Öl.

### Beispiel 5: Synthese von 2-(n-Butyl)-5-nitro-3(2H)-benzofuranon

In einem 50 ml-Kolben wurden 2.9 ml konzentrierte Schwefelsäure vorgelegt und auf 5°C gekühlt. Dann wurde 1.0 g 2-(n-Butyl)-3(2H)-benzofuranon langsam zugegeben, wobei sich eine bräunliche Färbung des Reaktionsgemisches ergab. Es wurde 15 Minuten gerührt. Anschließend wurde langsam 0.7 g Nitriersäure so zugetropft, dass die Temperatur nicht wesentlich über 5°C stieg. Danach wurde die Reaktionsmischung auf Eis gegossen, gerührt und mit Dichlormethan extrahiert. Die Dichlormethan-Extrakte wurden mit Wasser gewaschen, über Natriumsulfat getrocknet und im Vakuum bis zur Trockne eingeengt. Das so erhaltene Material (1.0 g) wies nach GC einen Gehalt von ca. 70 % (Flächenprozente) an 2-(n-Butyl)-5-nitro-3(2H)-benzofuranon auf. Eine weitere Reinigung kann durch Säulenchromatographie (Eluent: Hexan:Ethylacetat = 10:1) erfolgen.

¹H NMR (CDCl₃, 400 MHz): δ = 8.49 (d, *J*= 2.4 Hz, 1 H, Ar-H), 8.45 (dd, *J*_{*1*} = 9.0 Hz, *J*₂ = 2.4 Hz, 1 H, Ar-H), 7.17 (d, *J =* 9.0 Hz, 1 H, Aryl-H), 4.69 (dd, *J*_{*1*} *=* 8.0 Hz, *J*₂ = 4.4 Hz, 1 H, O=C-CH), 2.0 - 1.9 (2 mal m, 2 H, CH-*CH*_{*2*}), 1.40 (m, 2 H, CH₂), 1.33 (m, 2 H, CH₂), 0.85 (t, *J* = 7.2 Hz, 3 H, CH₃).

### Beispiel 6: Synthese von 2-(n-Butyl)-5-nitro-2,3-dihydro-benzofuran-3-ol

In einem 50 ml-Kolben wurden 700 mg 2-(n-Butyl)-5-nitro-3(2H)-benzofuranon in 10 ml Ethanol vorgelegt und auf 0°C gekühlt. Dazu wurde eine Lösung von 130 mg NaBH₄ in 5 ml Ethanol getropft, wobei sich sofort eine intensive Rotfärbung einstellte. Man ließ auf Raumtemperatur kommen und erhitzte anschließend kurzzeitig auf Rückfluss. Nach dem Abkühlen auf Raumtemperatur wurde das Reaktionsgemisch in Wasser und Dichlormethan aufgenommen, die organische Phase abgetrennt und die wässrige Phase nochmals mit Dichlormethan extrahiert. Nach Trocknung der vereinigten organischen Phasen über Natriumsulfat, Entfernen der Lösungsmittel im Vakuum und Säulenchromatographie (Eluent: Hexan:Ethylacetat = 5:1) wurden 400 mg (57 % d. Th.) 2-(n-Butyl)-5-nitro-2,3-dihydro-benzofuran-3-ol erhalten.

### Beispiel 7: Synthese von 2-(n-Butyl)-5-nitrobenzofuran

In einem 50 ml-Kolben wurden 300 mg 2-(n-Butyl)-5-nitro-2,3-dihydro-benzofuran-3-ol in Ethanol vorgelegt und 1 ml konzentrierte Schwefelsäure zugesetzt. Anschließend wurde 4 Stunden lang auf Rückfluss erhitzt. Nach dem Abkühlen setzte man 10 ml Wasser zu, destillierte das Ethanol ab und extrahierte mit Dichlormethan. Nach Waschen der organischen Phase mit Natriumhydrogencarbonatlösung, Trocknen über Natriumsulfat und Entfernen des Lösungsmittels im Vakuum wurden 220 mg (80 % d. Th.) 2-(n-Butyl)-5-nitrobenzofuran als gelbes Öl erhalten.

## Patentansprüche

1. Verbindungen der Formel (II), 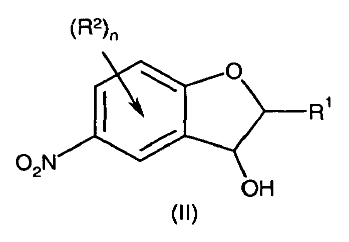 in der
• R¹ für Wasserstoff oder C₁-C₁₂-Alkyl steht und
• R² jeweils unabhängig steht für: Fluor, Chlor, Brom, Iod, C₁-C₁₂₋Alkyl, C₁-C₁₂-Alkoxy, Hydroxy, NR³R⁴ oder CONR³R⁴, wobei R³ und R⁴ jeweils unabhängig voneinander für Wasserstoff oder C₁-C₁₂-Alkyl steht oder NR³R⁴ zusammen für einen cyclischen Aminorest mit 4 bis 12 Kohlenstoffatomen steht, COO-(C₁-C₁₂-Alkyl), -COO(C₄-C₂₄₋Aryl), -COO(C₅-C₂₅-Arylalkyl), CO(C₁-C₁₂-Alkyl), CO(C₄-C₂₄-Aryl) oder C₁-C₁₂-Fluoralkyl und
• n für Null, eins zwei oder drei steht oder
für den Fall dass n zwei oder drei ist, zwei benachbarte Substituenten R² Teil eines annelierten Ringsystems sein können, das seinerseits gegebenenfalls mit den für R² vorstehend genannten Resten substituiert sein kann, wobei
• 2-(n-Butyl)-5-nitrobenzofuran ausgenommen ist.

2. 2-(n-Butyl)-5-nitro-2,3-dihydrobenzofuran-3-ol.

3. Verbindungen der Formel (III), 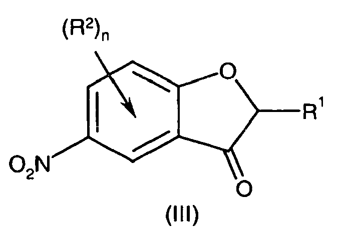 in der R² und n die unter der Formel (I) in Anspruch 1 genannte Bedeutung besitzen und R¹ für n-Butyl steht.

4. 2-(n-Butyl)-5-nitro-3(2H)-benzofuranon.

5. 3-Acetoxy-2-(n-butyl)-benzofuran.

6. 2-(1-Methoxycarbonyl-pentoxy)-benzoesäuremethylester, 2-(1-Methoxycarbonyl-pentoxy)-benzoesäureethylester, 2-(1-Ethoxycarbonyl-pentoxy)-benzoesäureethylester, 2-(1-Ethoxycarbonyl-pentoxy)-benzoesäuremethylester, 2-(1-Methoxycarbonyl-pentoxy)-benzoesäure, 2-(1-Ethoxycarbonylpentoxy)-benzoesäure, 2-(1-Carboxy-pentoxy)-benzoesäureethylester und 2-(1-Carboxy-pentoxy)-benzoesäuremethylester.

7. Verfahren zur Herstellung von Verbindungen der Formel (I), 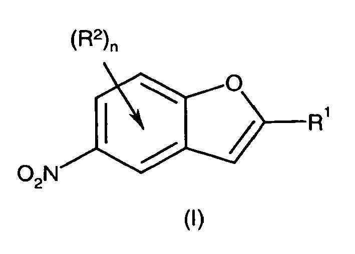 in der
• R¹ für Wasserstoff oder C₁-C₁₂-Alkyl und R² jeweils unabhängig stehen für: Fluor, Chlor, Brom, lod, C₁-C₁₂-Alkyl, C₁-C₁₂-Alkoxy, Hydroxy, NR³R⁴ oder CONR³R⁴, wobei R³ und R⁴ jeweils unabhängig voneinander für Wasserstoff oder C₁-C₁₂-Alkyl steht oder NR³R⁴ zusammen für einen cyclischen Aminorest mit 4 bis 12 Kohlenstoffatomen steht, COO-(C₁-C₁₂-Alkyl), -COO(C₄-C₂₄-Aryl), -COO(C₅-C₂₅-Arylalkyl), CO(C₁-C₁₂-Alkyl), CO(C₄-C₂₄-Aryl) oder C₁-C₁₂-Fluoralkyl und
• n für Null, eins zwei oder drei steht oder
für den Fall dass n zwei oder drei ist, zwei benachbarte Substituenten R² Teil eines annelierten Ringsystems sein können, das seinerseits gegebenenfalls mit den für R² vorstehend genannten Resten substituiert sein kann
**dadurch gekennzeichnet, dass** Verbindungen der Formel (II), 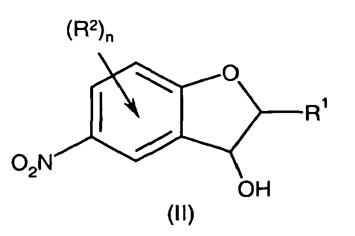 in der R¹, R² und n die unter der Formel (I) genannte Bedeutung besitzen,
durch Dehydratisierung in Verbindungen der Formel (I) überführt werden.

8. Verfahren gemäß Anspruch 7, **dadurch gekennzeichnet, dass** 2-(n-Butyl)-5-nitrobenzofuran hergestellt wird.

9. Verfahren gemäß mindestens einem der Ansprüche 7 und 9, **dadurch gekennzeichnet, dass** zur Dehydratisierung Protonensäuren oder Hydroxide eingesetzt werden.

10. Verfahren gemäß mindestens einem der Ansprüche 7 bis 9, **dadurch gekennzeichnet, dass** die Verbindungen der Formel (II) durch Reduktion von Verbindungen der Formel (III) erhalten werden, 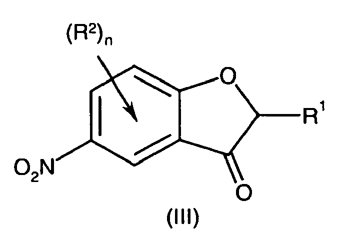 in der R¹, R² und n die unter der Formel (I) in Anspruch 10 genannte Bedeutung besitzen.

11. Verfahren gemäß Anspruch 10, **dadurch gekennzeichnet, dass** die Reduktion der Verbindungen der Formel (III) durch Aluminium-Wasserstoff- oder Bor-Wasserstoff-Verbindungen erfolgt.

12. Verfahren gemäß mindestens einem der Ansprüche 10 bis 11, **dadurch gekennzeichnet, dass** die Verbindungen der Formel (III) durch Nitrierung von Verbindungen der Formel (IV) erhalten werden, 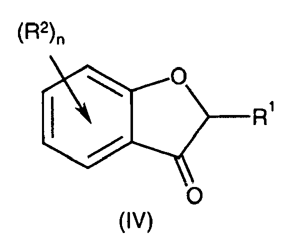 in der R¹, R² und n die unter der Formel (I) genannten Bedeutungen besitzen.

13. Verfahren gemäß Anspruch 12, **dadurch gekennzeichnet, dass** die Verbindungen der Formel (IV) durch Hydrolyse von Verbindungen der Formel (V) erhalten werden, 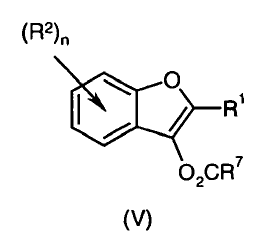 in der
R¹, R²und n die unter der Formel (I) in Anspruch 10 genannte Bedeutung besitzen und
R⁷ für C₁-C₁₂-Alkyl, C₅-C₂₅-Arylalkyl, C₄-C₂₄-Aryl oder C₁-C₁₂-Fluoralkyl steht.

14. Verfahren gemäß Anspruch 13, **dadurch gekennzeichnet, dass** die Verbindungen der Formel (V) durch Cyclisierung-Decarboxylierung von Verbindungen der Formel (VI), 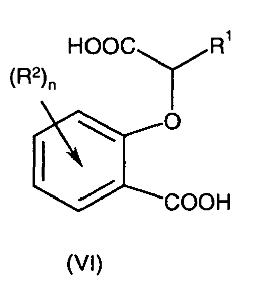 in der R¹, R² und n die unter der Formel (I) in Anspruch 10 genannte Bedeutung besitzen,
in Gegenwart mindestens einer Verbindung der Formel (RIII)
R⁷COR⁸ (RIII)
in der
R⁷ die unter der Formel (V) genannte Bedeutung besitzt und
R⁸ für -O₂CR⁷, Hydroxy oder OM steht, wobei M für ein Erdalkali- oder Alkalimetall steht,
hergestellt werden.

15. Verfahren gemäß Anspruch 14, **dadurch gekennzeichnet, dass** die Verbindungen der Formel (VI) durch Hydrolyse von Verbindungen der Formel (VII) erhalten werden, 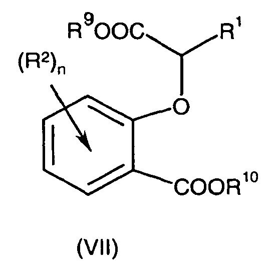 in der
R¹, R² und n die unter der Formel (I) genannte Bedeutung besitzen und
R⁹ und R¹⁰ jeweils unabhängig voneinander für Wasserstoff, C₁-C₁₂-Alkyl, C₅-C₂₅-Arylalkyl oder C₄-C₂₄-Aryl stehen.

16. Verfahren gemäß Anspruch 15, **dadurch gekennzeichnet, dass** die Verbindungen der Formel (VII) durch Umsetzung von Verbindungen der Formel (VIII), 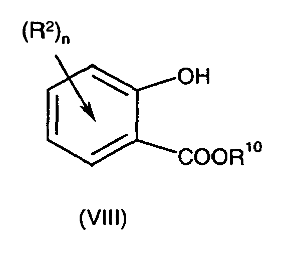 in der
R² und n die unter der Formel (I) in Anspruch 10 genannten Bedeutungen besitzen und
R¹⁰ die unter der Formel (VII) genannte Bedeutung besitzt
mit Verbindungen der Formel (IX) erhalten werden, 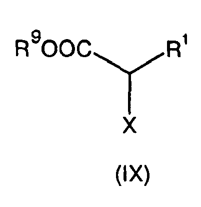 in der
R¹ die unter der Formel (I) in Anspruch 10 genannten Bedeutungen besitzt und
R⁹ die unter der Formel (VII) genannte Bedeutung besitzt und
X für Chlor, Brom, Iod oder R¹¹SO₃-steht, wobei
R¹¹ für C₁-C₁₂-Alkyl, C₄-C₂₄-Aryl, C₅-C₂₅-Arylalkyl oder C₁-C₁₂₋Fluoralkyl steht.

17. Verfahren gemäß Anspruch 14, **dadurch gekennzeichnet, dass** die Verbindungen der Formel (VI) durch Umsetzung von Verbindungen der Formel (VIII) mit Verbindungen der Formel (IX) unter gleichzeitig stattfindender Hydrolyse der Esterfunktionen in einer Eintopfreaktion hergestellt werden.

18. Verfahren zur Herstellung von Verbindungen der Formel (II), **dadurch gekennzeichnet, dass** es Reaktionsschritte gemäß mindestens einem der Ansprüche 16 bis 17 umfasst.

19. Verfahren zur Herstellung von Verbindungen der Formel (III), **dadurch gekennzeichnet, dass** es Reaktionsschritte gemäß mindestens einem der Ansprüche 12 bis 17 umfasst.

20. Verwendung von Verbindungen gemäß Ansprüchen 1 bis 6 oder Verbindungen, die gemäß mindestens einem der Ansprüche 7 bis 19 hergestellt wurden, zur Herstellung von Arzneimitteln und physiologisch aktiven Stoffen und zur Anwendung in einem Verfahren zur Herstellung von Arzneimitteln und physiologisch aktiven Stoffen.

21. Verwendung gemäß Anspruch 20, **dadurch gekennzeichnet, dass** Arzneimittel und physiologisch aktiven Stoffen solche sind, die zur Behandlung von Herzrhythmusstörungen eingesetzt werden.

22. Verwendung gemäß Anspruch 21, **dadurch gekennzeichnet, dass** der physiologisch aktive Stoff Dronedarone ist.

## Claims

1. Compounds of the formula (II), 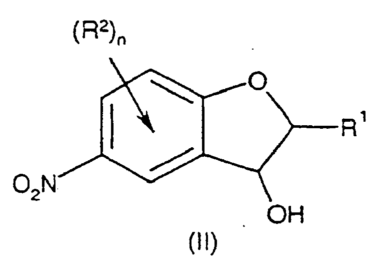 in which
• R¹ is hydrogen or C₁-C₁₂-alkyl, and
• R² are in each case independently of one another: fluorine, chlorine, bromine, iodine, C₁-C₁₂-alkyl, C₁-C₁₂-alkoxy, hydroxyl, NR³R⁴ or CONR³R⁴, where R³ and R⁴ are each, independently of one another, hydrogen or C₁-C₁₂-alkyl, or NR³R⁴ as a whole is a cyclic amino radical having 4 to 12 carbon atoms, COO-(C₁-C₁₂-alkyl), -COO(C₄-C₂₄-aryl), -COO(C₅-C₂₅-arylalkyl), CO(C₁-C₁₂-alkyl), CO(C₄-C₂₄-aryl) or C₁-C₁₂-fluoroalkyl and
• n is zero, one, two or three, or
in the case where n is two or three it is possible for two adjacent R² substituents to be part of a fused ring system which in turn may optionally be substituted by the radicals mentioned above for R²,
• 2-(n-butyl)-5-nitrobenzofuran being excluded.

2. 2-(n-Butyl)-5-nitro-2,3-dihydrobenzofuran-3-ol.

3. Compounds of the formula (III), 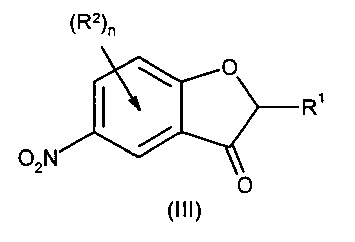 in which R² and n have the meaning specified under formula (I) in claim 1, and R¹ is n-butyl.

4. 2-(n-Butyl)-5-nitro-3(2H)-benzofuranone.

5. 3-Acetoxy-2-(n-butyl)-benzofuran.

6. Methyl 2-(1-methoxycarbonylpentoxy)benzoate, ethyl 2-(1-methoxycarbonylpentoxy)benzoate, ethyl 2-(1-ethoxycarbonylpentoxy)benzoate, methyl 2-(1-ethoxycarbonylpentoxy)benzoate, 2-(1-methoxycarbonylpentoxy)benzoic acid, 2-(1-ethoxycarbonylpentoxy)benzoic acid, ethyl 2-(1-carboxypentoxy)benzoate and methyl 2-(1-carboxypentoxy)benzoate.

7. Process for preparing compounds of the formula (I), 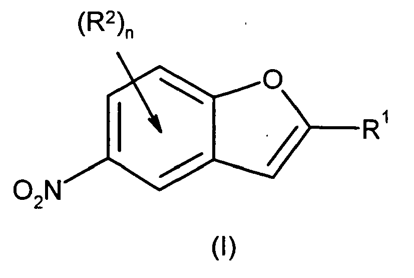 in which
• R¹ is hydrogen or C₁-C₁₂-alkyl, and R² are in each case independently: fluorine, chlorine, bromine, iodine, C₁-C₁₂-alkyl, C₁₋C₁₂-alkoxy, hydroxyl, NR³R⁴ or CONR³R⁴, where R³ and R⁴ are each, independently of one another, hydrogen or C₁-C₁₂-alkyl, or NR³R⁴ as a whole is a cyclic amino radical having 4 to 12 carbon atoms, COO-(C₁-C₁₂-alkyl), -COO(C₄-C₂₄-aryl), -COO(C₅-C₂₅₋arylalkyl), CO(C₁-C₁₂-alkyl), CO(C₄-C₂₄-aryl) or C₁-C₁₂-fluoroalkyl and
• n is zero, one, two or three, or
in the case where n is two or three it is possible for two adjacent R² substituents to be part of a fused ring system which in turn may optionally be substituted by the radicals mentioned above for R²,
**characterized in that** compounds of the formula (II) 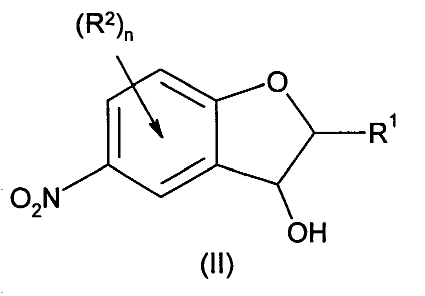 in which R¹, R² and n have the meaning under formula (I),
are converted by dehydration into compounds of the formula (I).

8. Process according to Claim 7 **characterized in that** 2-(n-butyl)-5-nitrobenzofuran is prepared.

9. Process according to at least one of claims 7 and 8, **characterized in that** protic acids or hydroxides are employed for the dehydration.

10. Process according to at least one of Claims 7 to 9, **characterized in that** the compounds of the formula (II) are obtained by reducing compounds of the formula (III) 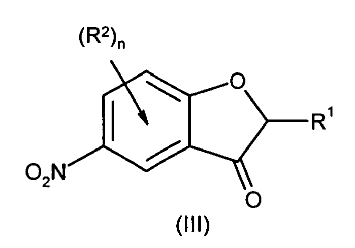 in which R¹, R² and n have the meaning specified under formula (I) in Claim 10.

11. Process according to Claim 10, **characterized in that** the compounds of the formula (III) are reduced by aluminium-hydrogen or boron-hydrogen compounds.

12. Process according to at least one of Claims 10 to 11, **characterized in that** the compounds of the formula (III) are obtained by nitrating compounds of the formula (IV) 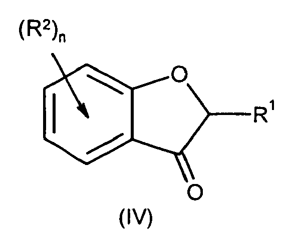 in which R¹, R² and n have the meanings specified under formula (I).

13. Process according to Claim 12, **characterized in that** the compounds of the formula (IV) are obtained by hydrolysing compounds of the formula (V) 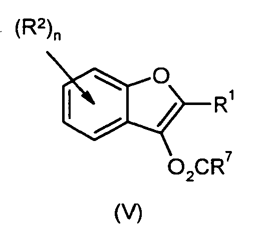 in which
R¹, R² and n have the meaning specified under formula (I) in Claim 10, and
R⁷ is C₁-C₁₂-alkyl, C₅-C₂₅-arylalkyl, C₄-C₂₄-aryl or C₁-C₁₂-fluoroalkyl.

14. Process according to Claim 13, **characterized in that** the compounds of the formula (V) are obtained by cyclizing decarboxylation of compounds of the formula (VI), 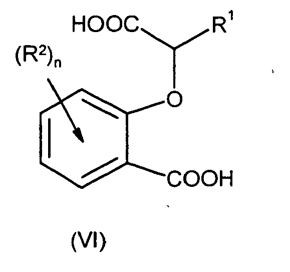 in which R¹, R² and n have the meaning specified under formula (I) in Claim 10,
in the presence of at least one compound of the formula (RIII)
R⁷COR⁸ (RIII)
in which
R⁷ has the meaning specified under formula (V), and
R⁸ is -O₂CR⁷, hydroxyl or OM, where M is an alkaline earth metal or alkali metal.

15. Process according to Claim 14, **characterized in that** the compounds of the formula (VI) are obtained by hydrolysing compounds of the formula (VII) 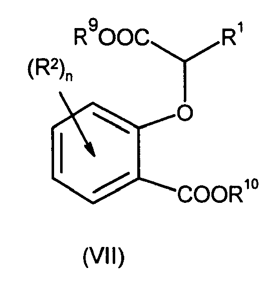 in which
R¹, R² and n have the meaning specified under formula (I), and
R⁹ and R¹⁰ are each independently of one another hydrogen, C₁-C₁₂-alkyl, C₅-C₂₅-arylalkyl or C₄-C₂₄-aryl.

16. Process according to Claim 15, **characterized in that** the compounds of the formula (VII) are obtained by reacting compounds of the formula (VIII) 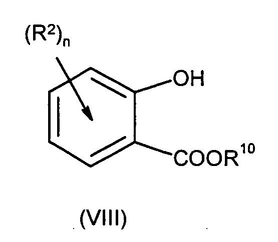 in which
R² and n have the meaning specified under formula (I) in Claim 10 and
R¹⁰ has the meaning specified under formula (VII),
with compounds of the formula (IX) 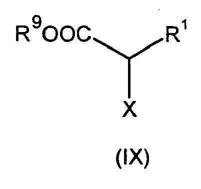 in which
R¹ has the meanings specified under formula (I) in Claim 10, and
R⁹ has the meaning specified under formula (VII), and
R¹¹ is C₁-C₁₂-alkyl, C₄-C₂₄-aryl, C₅-C₂₅-arylalkyl or C₁-C₁₂₋fluoroalkyl.

17. Process according to Claim 14, **characterized in that** the compounds of the formula (VI) are prepared by reacting compounds of the formula (VIII) with compounds of the formula (IX) in a one-pot reaction with hydrolysis of the ester functions taking place simultaneously.

18. Process for preparing compounds of the formula (II), **characterized in that** it comprises reaction steps according to at least one of Claims 16 to 17.

19. Process for preparing compounds of the formula (III), **characterized in that** it comprises reaction steps according to at least one of Claims 12 to 17.

20. Use of compounds according to Claims 1 to 6 or compounds which have been prepared according to at least one of Claims 7 to 19 for producing medicaments and physiologically active substances and for use in a process for producing medicaments and physiologically active substances.

21. Use according to Claim 20, **characterized in that** medicaments and physiologically active substances are those employed for the treatment of cardiac arrhythmias.

22. Use according to Claim 21, **characterized in that** the physiologically active substance is dronedarone.

## Revendications

1. Composés de formule (II), 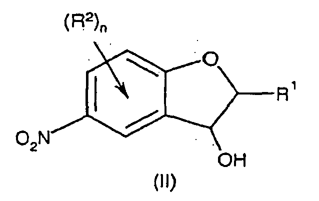 dans laquelle
• R¹ représente un hydrogène ou un alkyle en C₁-C₁₂, et
• R² représente chacun indépendamment : un fluor, un chlore, un brome, un iode, un alkyle en C₁-C₁₂, un alcoxy en C₁-C₁₂, un hydroxy, un NR³R⁴ ou un CONR³R⁴, dans lesquels R³ et R⁴ représentent chacun, indépendamment l'un de l'autre, un hydrogène ou un alkyle en C₁-C₁₂, ou NR³R⁴ représente conjointement un radical amino cyclique ayant de 4 à 12 atomes de carbone, un COO-(C₁-C₁₂-alkyle), un -COO(C₄-C₂₄-aryle), un -COO(C₅-C₂₅-arylalkyle), un CO(C₁-C₁₂-alkyle), un CO(C₄-C₂₄-aryle) ou un fluoroalkyle en C₁-C₁₂, et
• n vaut zéro, un, deux ou trois, ou
au cas où n vaut deux ou trois, deux substituants R² voisins peuvent être une partie d'un système cyclique condensé, qui peut, pour sa part, être éventuellement substitué par les radicaux mentionnés ci-dessus pour R², où
• le 2-(n-butyl)-5-nitrobenzofurane est exclu.

2. 2-(n-Butyl)-5-nitro-2,3-dihydrobenzofurane-3-ol.

3. Composés de formule (III), 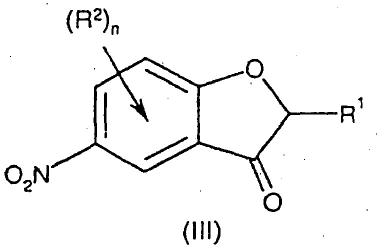 dans laquelle R² et n présentent la signification mentionnée dans la revendication 1 pour la formule (I) et R¹ représente un n-butyle.

4. 2-(n-Butyl)-5-nitro-3(2H)-benzofuranone.

5. 3-Acétoxy-2-(n-butyl)-benzofurane.

6. Ester méthylique de l'acide 2-(1-méthoxycarbonylpentoxy)benzoïque, ester éthylique de l'acide 2-(1-méthoxycarbonyl-pentoxy)benzoïqué, ester éthylique de l'acide 2-(1-éthoxycarbonyl-pentoxy)-benzoïque, ester méthylique de l'acide 2-(1-éthoxycarbonyl-pentoxy)benzoïque, acide 2-(1-méthoxycarbonyl-pentoxy)benzoïque, acide 2-(1-éthoxycarbonyl-pentoxy) benzoïque, ester éthylique de l'acide 2-(1-carboxy-pentoxy)benzoïque et ester méthylique de l'acide 2-(1-carboxy-pentoxy)-benzoïque.

7. Procédé de préparation de composés de formule (I), 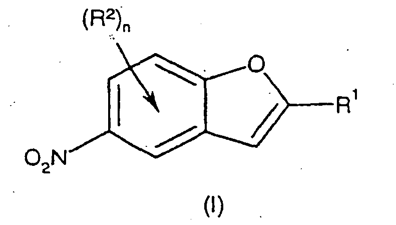 dans laquelle
• R¹ représente un hydrogène ou un alkyle en C₁-C₁₂, et R² représente chacun indépendamment : un fluor, un chlore, un brome, un iode, un alkyle en C₁-C₁₂, un alcoxy en C₁-C₁₂, un hydroxy, un NR³R⁴ ou un CONR³R⁴, dans lesquels R³ et R⁴ représentent chacun, indépendamment l'un de l'autre, un hydrogène ou un alkyle en C₁-C₁₂, ou NR³R⁴ représente conjointement un radical amino cyclique ayant de 4 à 12 atomes de carbone, un COO-(C₁-C₁₂-alkyle), un -COO(C₄-C₂₄-aryle), un -COO(C₅-C₂₅-arylalkyle), un CO(C₁-C₁₂-alkyle), un CO(C₄-C₂₄-aryle) ou un fluoroalkyle en C₁-C₁₂, et
• n vaut zéro, un, deux ou trois, ou
au cas où n vaut deux ou trois, deux substituants R² voisins peuvent être une partie d'un système cyclique condensé, qui peut, pour sa part, être éventuellement substitué par les radicaux mentionnés ci-dessus pour R²,
**caractérisé en ce que** des composés de formule (II), 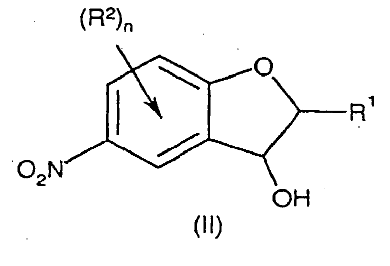 dans laquelle R¹, R² et n présentent la signification mentionnée pour la formule (I),
sont transformés en composés de formule (I) par déshydratation.

8. Procédé selon la revendication 7, **caractérisé en ce que** l'on prépare du 2-(n-butyl)-5-nitrobenzofurane.

9. Procédé selon au moins l'une des revendications 7 et 9, **caractérisé en ce que** l'on utilise des acides protoniques ou des hydroxydes pour la déshydratation.

10. Procédé selon au moins l'une des revendications 7 à 9, **caractérisé en ce que** les composés de formule (II) sont obtenus par réduction de composés de formule (III), 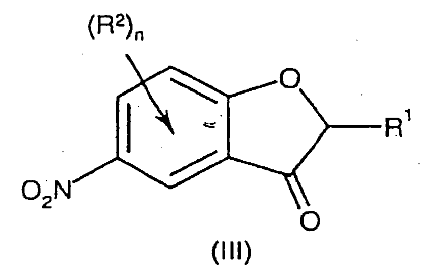 dans laquelle R¹, R² et n présentent la signification mentionnée dans la revendication 10 pour la formule (I).

11. Procédé selon la revendication 10, **caractérisé en ce que** la réduction des composés de formule (III) est effectuée par des composés d'aluminium-hydrogène ou de bore-hydrogène.

12. Procédé selon au moins l'une des revendications 10 à 11, **caractérisé en ce que** les composés de formule (III) sont obtenus par nitration de composés de formule (IV), 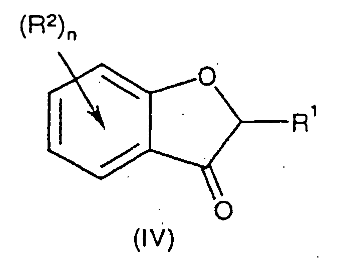 dans laquelle R¹, R² et n présentent les significations mentionnées pour la formule (I).

13. Procédé selon la revendication 12, **caractérisé en ce que** les composés de formule (IV) sont obtenus par hydrolyse de composés de formule (V), 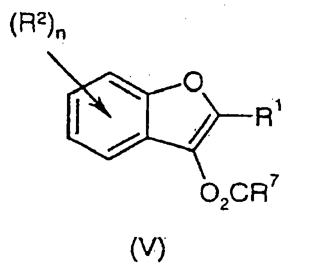 dans laquelle
R¹, R² et n présentent la signification mentionnée dans la revendication 10 pour la formule (I), et
R⁷ représente un alkyle en C₁-C₁₂, un C₅-C₂₅₋arylalkyle, un aryle en C₄-C₂₄ ou un fluoroalkyle en C₁-C₁₂.

14. Procédé selon la revendication 13, **caractérisé en ce que** les composés de formule (V) sont préparés par cyclisation-décarboxylation de composés de formule (VI), 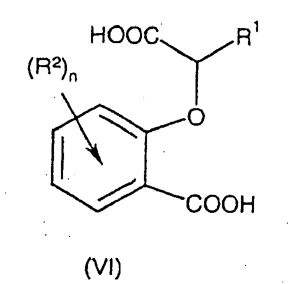 dans laquelle R¹, R² et n présentent la signification mentionnée dans la revendication 10 pour la formule (I),
en présence d'au moins un composé de formule (RIII)
R⁷COR⁸ (RIII)
dans laquelle
R⁷ présente la signification mentionnée pour la formule (V), et
R⁸ représente un -O₂CR⁷, un hydroxy ou un OM, dans lequel M représente un métal alcalino-terreux ou alcalin.

15. Procédé selon la revendication 14, **caractérisé en ce que** les composés de formule (VI) sont obtenus par hydrolyse de composés de formule (VII), 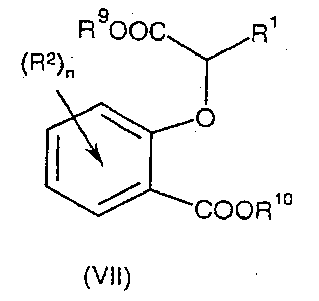 dans laquelle
R¹, R² et n présentent la signification mentionnée pour la formule (1), et
R⁹ et R¹⁰ représentent chacun, indépendamment l'un de l'autre, un hydrogène, un alkyle en C₁-C₁₂, un C₅-C₂₅-arylalkyle ou un aryle en C₄-C₂₄.

16. Procédé selon la revendication 15, **caractérisé en ce que** les composés de formule (VII) sont obtenus par réaction de composés de formule (VIII), 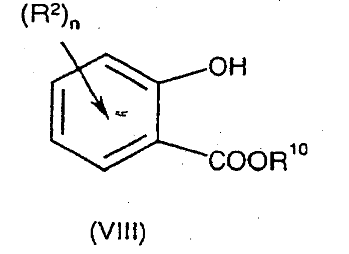 dans laquelle
R² et n présentent les significations mentionnées dans la revendication 10 pour la formule (I), et
R¹⁰ présente la signification mentionnée pour la formule (VII),
avec des composés de formule (IX), 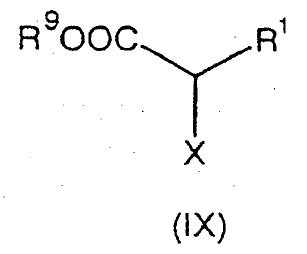 dans laquelle
R¹ présente les significations mentionnées dans la revendication 10 pour la formule (I), et
R⁹ présente la signification mentionnée pour la formule (VII), et
X représente un chlore, un brome, un iode ou un R¹¹SO₃-, dans lequel
R¹¹ représente un alkyle en C₁-C₁₂, un aryle en C₄-C₂₄, un C₅-C₂₅-arylalkyle ou un fluoralkyle en C₁-C₁₂.

17. Procédé selon la revendication 14, **caractérisé en ce que** les composés de formule (VI) sont préparés par réaction de composés de formule (VIII) avec des composés de formule (IX) avec hydrolyse simultanée de la fonction ester dans une réaction en récipient unique.

18. Procédé de préparation de composés de formule (II), **caractérisé en ce qu'**il comprend des étapes de réaction selon au moins l'une des revendications 16 à 17.

19. Procédé de préparation de composés de formule (III), **caractérisé en ce qu'**il comprend des étapes de réaction selon au moins l'une des revendications 12 à 17.

20. Utilisation de composés selon les revendications 1 à 6 ou de composés qui ont été préparés selon au moins l'une des revendications 7 à 19, pour la production de médicaments et de substances physiologiquement actives et pour une application dans un procédé de production de médicaments et de substances physiologiquement actives.

21. Utilisation selon la revendication 20, **caractérisée en ce que** les médicaments et les substances physiologiquement actives sont ceux qui sont utilisés pour le traitement de troubles du rythme cardiaque.

22. Utilisation selon la revendication 21, **caractérisée en ce que** la substance physiologiquement active est la dronédarone.
